(19) Europäisches Patentamt
European Patent Office
Office européen des brevets

(11) Veröffentlichungsnummer: **0 155 238**
**A2**

(12) # EUROPÄISCHE PATENTANMELDUNG

(21) Anmeldenummer: **85810104.1**

(22) Anmeldetag: **11.03.85**

(51) Int. Cl.⁴: **C 07 D 303/27**, C 07 D 303/36, C 07 D 303/16, C 07 D 301/27 // C07C43/23, C07C69/757, C07C91/40

(30) Priorität: **15.03.84 CH 1288/84**

(43) Veröffentlichungstag der Anmeldung: **18.09.85 Patentblatt 85/38**

(84) Benannte Vertragsstaaten: **CH DE FR GB IT LI NL**

(71) Anmelder: **CIBA-GEIGY AG, Postfach, CH-4002 Basel (CH)**

(72) Erfinder: **Fiaux, André, Dr., Chemin sous l'Eglise, CH-1867 Ollon (CH)**
Erfinder: **Nguyen, Dinh Ly, Dr., 10463 Slater Av. No. 103, Fountain Valley, CA 92708 (US)**

(54) **Verfahren zur Herstellung bestimmter Glycidylverbindungen.**

(57) Zweistufiges Verfahren zur Herstellung von Glycidylverbindungen mit geringem Gehalt an hydrolysierbarem Halogen durch Umsetzung von chlor- oder bromsubstituierten Bisphenolen, Mono- oder Dicarbonsäuren, Mono- oder Diaminen oder von Cyanursäure mit einem Epihalogenydrin, wobei auf 1 Äquivalent reaktives H-Atom mehr als 1 und höchstens 1,8 Äquivalente Epihalogenhydrin eingesetzt werden, die in erster Stufe erhaltenen Halogenhydrinverbindungen mittels azeotroper Destillation vom überschüssigen Epihalogenhydrin befreit und dann durch sogenanntes Strippen mit Wasser gewaschen werden, und in zweiter Stufe die Dehydrohalogenierung der Halogenhydrinverbindungen in einem inerten, im wesentlichen mit Wasser nicht mischbaren organischen Lösungsmittel vorgenommen wird.

EP 0 155 238 A2

CIBA-GEIGY AG
Basel (Schweiz)

3-14793/+

Verfahren zur Herstellung bestimmter Glycidylverbindungen

Die Erfindung betrifft ein Verfahren zur Herstellung bestimmter Glycidylverbindungen nach dem Zweistufenverfahren unter Isolierung und Reinigung der nach der ersten Stufe erhaltenen Halogenhydrinverbindungen.

Glycidylverbindungen, wie beispielsweise Glycidyläther von Phenolen oder Alkoholen, Glycidylester von Carbonsäuren oder N-Glycidylverbindungen von Aminen, lassen sich durch Umsetzung von Epihalogenhydrin mit den entsprechenden Phenolen, Alkoholen, Carbonsäuren bzw. Aminen bekanntlich ein- oder zweistufig herstellen.

Beim zweistufigen Verfahren wird, vorzugsweise zur Herstellung von niedermolekularen Glycidylverbindungen, das in erster Stufe normalerweise im Ueberschuss eingesetzte Epihalogenhydrin, wie beispielsweise Epichlorhydrin, vor der in zweiter Stufe mit wässriger Alkalilösung vorgenommenen Dehydrohalogenierungsreaktion entweder aus der Reaktionslösung entfernt oder es wird sowohl als Dehydrohalogenierungsmittel und als Lösungsmittel in der Reaktionslösung belassen, um das durch die Natronlauge zugeführte und das bei der Umsetzungsreaktion entstehende Wasser azeotrop aus dem Reaktionsgemisch zu entfernen.

Zur Herstellung von reinen, niedermolekularen Glycidylverbindungen mit geringem Gehalt an hydrolysierbarem Halogen sind bereits viele Verfahrensverbesserungen, wie zum Beispiel Verwendung bestimmter

Lösungsmittel oder spezifischer Katalysatoren, vorgeschlagen worden, wie sie beispielsweise in "Handbook of Epoxy Resins" von H. Lee und K. Neville, Ausgabe 1967, Kapitel 2, zusammenfassend beschrieben werden.

Im US-Patent 4,373,073 wird ferner ein zweistufiges Verfahren zur Herstellung von Glycidyläthern durch Umsetzung von Phenolen mit einem grossen stöchiometrischen Ueberschuss an Epichlorhydrin in Gegenwart eines Katalysators beschrieben, wobei der in erster Stufe entstehende Chlorhydrinäther durch Entfernen des überschüssigen Epichlorhydrins isoliert wird und in zweiter Stufe die Dehydrohalogenierung des Chlorhydrinäthers mittels Alkalilauge und in Gegenwart eines zweiten spezifischen Katalysators vorgenommen wird.

Es wurde nun gefunden, dass sich bestimmte Glycidylverbindungen mit geringem Gehalt an hydrolisierbarem Halogen in einfacherer und ökonomischerer Weise nach dem zweistufigen Verfahren herstellen lassen, wenn man das Epihalogenhydrin nur im geringen stöchiometrischen Ueberschuss einsetzt, die nach der ersten Stufe erhaltenen Halogenhydrinverbindungen isoliert und mit Wasser reinigt. Ferner erübrigt sich beim erfindungsgemässen Verfahren für die zweite Verfahrensstufe die Zugabe eines Katalysators.

Gegenstand vorliegender Erfindung ist somit ein Verfahren zur Herstellung von Glycidyläthern der Formel I

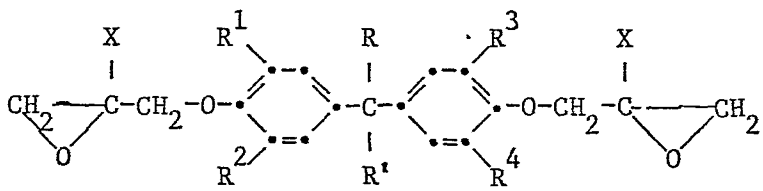

(I) ,

worin R und R' unabhängig voneinander je ein Wasserstoffatom, Alkyl

mit 1 bis 15 C-Atomen, Phenyl, Cyclohexyl oder Cyclopentyl, $R^1$, $R^2$, $R^3$ und $R^4$ unabhängig voneinander je ein Chlor- oder Bromatom und beide X je ein H-Atom oder Methyl bedeuten, von Glycidylestern der Formel II

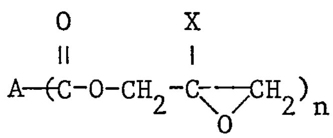 (II) ,

worin n 1 oder 2 bedeutet, A, wenn n 1 ist, für einen einwertigen aliphatischen Rest oder, wenn n 2 ist, für eine direkte Bindung oder einen zweiwertigen aliphatischen, aromatischen oder cycloaliphatischen Rest und X für ein H-Atom oder Methyl stehen, von N-Glycidylverbindungen der Formel III

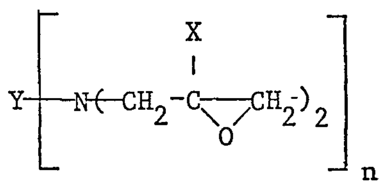 (III) ,

worin n 1 oder 2 bedeutet, Y für einen n-wertigen aliphatischen oder aromatischen Rest und X für ein H-Atom oder Methyl stehen, von Triglycidylisocyanurat oder Tri-(β-methylglycidyl)-isocyanurat durch Umsetzung eines Epihalogenhydrins mit einer Verbindung der Formeln Ia, IIa oder IIIa

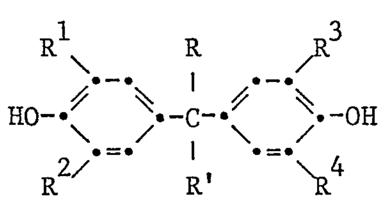 (Ia) ,

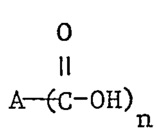 (IIa) , $Y-(NH_2)_n$ (IIIa)

oder mit Cyanursäure, gegebenenfalls im wässrigen Medium und/oder in Gegenwart eines in Wasser beständigen Katalysators, zu den entsprechenden Halogenhydrinverbindungen und anschliessende Dehydrohalogenierung mit wässrigem Alkali zu den Glycidylverbindungen der Formeln I, II bzw. III oder zu Triglycidyl- bzw. Tri-(β-methylglycidyl)-isocyanurat, dadurch gekennzeichnet, dass man auf 1 Aequivalent reaktives H-Atom in den Verbindungen der Formeln Ia bis IIIa oder in der Cyanursäure mehr als 1 und höchstens 1,8 Aequivalente Epihalogenhydrin einsetzt, die in erster Stufe erhaltenen Halogenhydrinverbindungen mittels azeotroper Destillation vom überschüssigen Epihalogenhydrin befreit und dann durch sogenanntes Strippen mit Wasser wäscht und in zweiter Stufe die Dehydrohalogenierung der Halogenhydrinverbindungen in einem inerten, im wesentlichen mit Wasser nicht mischbaren organischen Lösungsmittel vornimmt.

Vorzugsweise setzt man beim erfindungsgemässen Verfahren Verbindungen der Formeln Ia, IIa oder IIIa ein, insbesondere der Formel Ia oder IIa.

Als Epihalogenhydrin eignen sich zum Beispiel Epichlorhydrin, β-Methylepichlorhydrin, Epibromhydrin, β-Methylepibromhydrin oder Glycerindichlorhydrin. Vorzugsweise verwendet man beim erfindungsgemässen Verfahren Epichlorhydrin.

Verbindungen der Formel Ia sind beispielsweise Bis-(3,5-dibrom-4-hydroxyphenyl)-methan, 2,2-Bis-(3,5-dibrom-4-hydroxyphenyl)-propan, 1,1-Bis-(3,5-dichlor-4-hydroxyphenyl)-äthan oder 2,2-Bis-(3,5-dichlor-4-hydroxyphenyl)-propan. Vorzugsweise setzt man beim erfindungsgemässen Verfahren als Verbindungen der Formel Ia die Tetrabromverbindungen ein.

Als geeignete Verbindungen der Formel IIa mit der Bedeutung von n gleich 1 seien beispielsweise Essigsäure, Acryl- oder Methacrylsäure, Propionsäure, Laurinsäure und Stearinsäure genannt. Als Verbindungen der Formel IIa mit der Bedeutung von n gleich 2 sind beispielsweise von den aliphatischen Dicarbonsäuren, Oxalsäure, Bernsteinsäure, Glutarsäure, Adipinsäure, 2,2,4-Trimethyl- und 2,4,4-Trimethyladipinsäure sowie beide Isomeren enthaltende Gemische, Sebazinsäure, Fumarsäure, Maleinsäure und dimerisierte Linolsäure,

von den aromatischen Dicarbonsäuren, Phthalsäure, Isophthalsäure, Terephthalsäure, 2,5-Dimethylterephthalsäure, Naphthalin-2,6-dicarbonsäure, Naphthalin-1,8-dicarbonsäure, Naphthalin-2,3-dicarbonsäure, Diphenyläther-4,4'-dicarbonsäure, Diphenyl-4,4'-dicarbonsäure und Diphenyl-2,2'-dicarbonsäure, Tetrachlorphthalsäure und 2,5-Dichlorterephthalsäure, und von den cycloaliphatischen Dicarbonsäuren, Tetrahydrophthalsäure, Methyl-tetrahydrophthalsäure, isomerisierte 4-Methyl-tetrahydrophthalsäure, Endomethylen-tetrahydrophthalsäure, Hexahydrophthalsäure, Methylhexahydrophthalsäure, Endomethylen-hexahydrophthalsäure, Hexahydroterephthalsäure und Hexahydroisophthalsäure genannt.

Vorzugsweise setzt man beim erfindungsgemässen Verfahren als Verbindungen der Formel IIa die Dicarbonsäuren ein, insbesondere die cycloaliphatischen Dicarbonsäuren.

Beispiele für geeignete Monoamine der Formel IIIa sind n-Butylamin, Aethanolamin, 2-Aethylhexylamin, 2-Phenyläthylamin, 2-Butoxyäthylamin, n-Octylamin, Benzylamin und Anilin. Als geeignete Diamine der Formel IIIa sind beispielsweise m-Xylylendiamin, Bis-(4-aminophenyl)-methan, 4,4'-Diaminodiphenyl, Bis-(3-aminophenyl)-sulfon, Bis-(4-aminophenyl)-sulfon, 1,2-Bis-(4-aminophenyl)-äthan, 2,2-Bis-(4-aminophenyl)-propan und 2,2-Bis-(3,5-dibrom-4-aminophenyl)-propan genannt.

Vorzugsweise setzt man beim erfindungsgemässen Verfahren als Verbindungen der Formel IIIa die Diamine ein, insbesondere solche, die der Formel IV entsprechen

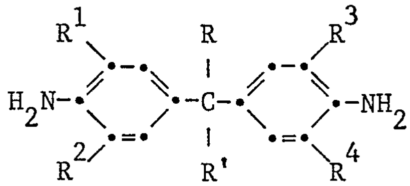

(IV) ,

worin R, R', $R^1$, $R^2$, $R^3$ und $R^4$ die gleiche Bedeutung wie in Formel I haben und $R^1$ bis $R^4$ ausserdem je für ein Wasserstoffatom stehen.

Beim erfindungsgemässen Verfahren werden das Epihalogenhydrin und die Verbindungen der Formeln Ia bis IIIa und die Cyanursäure in solchen Mengen eingesetzt, dass in der Reaktionslösung ein geringer stöchiometrischer Ueberschuss an Epihalogenhydrin vorliegt, dass heisst, auf 1 Aequivalent reaktives H-Atom in den Verbindungen der Formeln Ia bis IIIa und der Cyanursäure werden mehr als 1 und bis zu 1,8 Aequivalente Epihalogenhydrin, vorzugsweise 1,05 bis 1,5 Aequivalente Epihalogenhydrin, eingesetzt.

Das erfindungsgemässe Verfahren wird in erster Stufe vorzugsweise im wässrigen Medium durchgeführt, wobei die Menge des dem Reaktionsgemisch zugesetzten Wassers an sich nicht kritisch ist, doch vorteilhaft nur so viel beträgt, dass ein gut rührbares Reaktionsgemisch vorliegt.

Die Umsetzung des Epihalogenhydrins mit den Verbindungen der Formeln Ia bis IIIa und der Cyanursäure wird vorzugsweise in Gegenwart eines in Wasser beständigen Katalysators durchgeführt, wobei man bevorzugt alkalische Katalysatoren einsetzt.

Als alkalische Katalysatoren verwendet man beispielsweise Phasenumwandlungskatalysatoren, wie quaternäre Ammoniumsalze, beispielsweise Tetramethylammoniumchlorid, Tetraäthylammoniumchlorid, Benzyltrimethylammoniumchlorid, Benzyltrimethylammoniumacetat, Methyltriäthylammoniumchlorid, Tetrabutylammoniumchlorid oder Tetrabutylammoniumsulfat, quaternäre Ammoniumbasen, beispielsweise Benzyltrimethylammoniumhydroxid, sowie Kronenäther, beispielsweise 12-Kronen-4-äther (1,4,7,10-Tetraoxacyclododecan), 15-Kronen-5-äther (1,4,7,10,13-Pentaoxacyclopentadecan), 18-Kronen-6-äther oder Dibenzo-18-kronen-6-äther. Ferner eignen sich als Katalysatoren tertiäre Amine, wie beispielsweise 2,4,6-Tris-(dimethylaminomethyl)-phenol, Benzyldimethylamin, 1-Methylimidazol, 2-Aethyl-4-methylimidazol oder Aminopyridin.

Es ist vorteilhaft, die Umsetzungsreaktion bei erhöhter Temperatur

zwischen 50 und 95°C, vorzugsweise zwischen 70 und 95°, unter Rühren der Reaktionslösung vorzunehmen. Die Umsetzungsreaktion ist im allgemeinen nach etwa einer Stunde beendet. Zum Nachreagieren wird die Reaktionslösung zweckmässigerweise anschliessend noch etwa eine Stunde unter leichtem Rückfluss gehalten.

Nach der destillativen Entfernung des nicht umgesetzten Epihalogenhydrins und sonst flüchtiger Bestandteile wird die zurückbleibende Halogenhydrinverbindung mit Wasser durch sogenanntes Strippen weitergereinigt, indem man die Halogenhydrinverbindung in Wasser aufschlämmt und die restlichen flüchtigen Anteile mit Wasser im Vakuum destillativ entfernt. Dieser Vorgang, wobei Wasser als Schleppmittel verwendet wird, kann nötigenfalls mehrmals wiederholt werden.

Die auf diese Weise gereinigten Halogenhydrinverbindungen werden danach in einem inerten, im wesentlichen mit Wasser nicht mischbaren organischen Lösungsmittel mit wässrigen Alkalien dehydrohalogeniert.

Als inerte, im wesentlichen mit Wasser nicht mischbare organische Lösungsmittel eignen sich beispielsweise Ketone, wie Methyläthylketon, oder Methylisobutylketon, aromatische Kohlenwasserstoffe, wie Benzol, Toluol oder Xylol, oder auch aliphatische Kohlenwasserstoffe, wie Hexan, Heptan oder Octan.

Zur Dehydrohalogenierung werden in der Regel starke Alkalien verwendet. Vorzugsweise setzt man wässrige Natronlauge ein, doch können auch andere alkalische Reagentien, wie Kaliumhydroxid, Bariumhydroxid, Calciumhydroxid, Natriumcarbonat oder Kaliumcarbonat Verwendung finden. Vorzugsweise verwendet man beim erfindungsgemässen Verfahren 20 bis 100 gewichts-%ige Natronlauge. Im allgemeinen arbeitet man bei der Dehydrohalogenierung mit stöchiometrischen Mengen, doch ist es zweckmässig, die Halogenwasserstoffabspaltung unter Verwendung eines bis zu 20 %igen Ueberschusses über die stöchiometrisch erforderliche Menge an Alkali durchzuführen, bezogen auf den Gehalt an hydrolysierbarem Halogen in der Halogenhydrinverbindung.

Da bei der Dehydrohalogenierungsreaktion ein Zweiphasensystem vorliegt, ist es zweckmässig, die Reaktionslösung kräftig zu rühren. Es ist ferner vorteilhaft, die Dehydrohalogenierung bei erhöhter Temperatur, etwa zwischen 40 und 90°C, vorzunehmen. Die Dehydrohalogenierung kann unter Normaldruck oder unter azeotropen Bedingungen durchgeführt werden.

Nach Beendigung der Dehydrohalogenierungsreaktion wird die organische, das Epoxidharz enthaltende Phase von der wässrigen Phase abgetrennt und vorzugsweise mittels azeotroper Destillation vom organischen Lösungsmittel befreit. Vorzugsweise wird das zurückbleibende Epoxidharz ebenfalls mit Wasser gestrippt.

Die nach dem erfindungsgemässen Verfahren erhaltenen Epoxidharze zeichnen sich durch einen relativ niedrigen Gehalt an hydrolysierbarem Halogen aus und sind aufgrund ihrer verminderten Korrosionswirkung auf Metalle als Isolierharze für die Elektroindustrie besonders gut geeignet.

Die nach dem erfindungsgemässen Verfahren in erster Stufe isolierten Halogenhydrinverbindungen sind zum Teil bekannte Verbindungen. Soweit es sich um neue Verbindungen handelt, sind diese ebenfalls Gegenstand vorliegender Erfindung. Insbesondere sind die Di-(halogenhydrinäther) von chlor- und/oder bromsubstituierten Bisphenolen als reine, isolierte Verbindungen noch nicht in der Literatur beschrieben worden. Die Erfindung betrifft somit auch die Di-(halogenhydrinäther) der Formel V

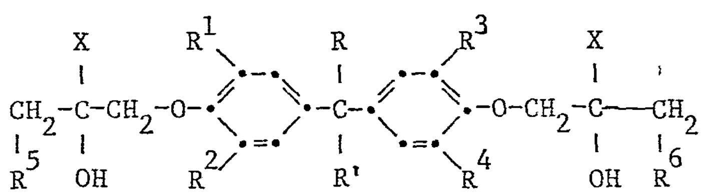

(V) ,

worin R, R', $R^1$, $R^2$, $R^3$, $R^4$ und beide X die gleiche Bedeutung wie in Formel I haben und $R^5$ und $R^6$ je für ein Brom- oder Chloratom stehen.

Die nach dem erfindungsgemässen Verfahren erhaltenen Verbindungen der Formel V stellen wertvolle Zwischenverbindungen dar, aus denen durch Dehydrohalogenierung Glycidylverbindungen erhalten werden, die weitgehend von Verunreinigungen frei sind, insbesondere von dem Diglycidyläther, 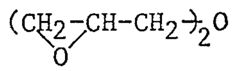 , dem toxische Eigenschaften zugeschrieben werden.

In den folgenden Beispielen steht % für Gewichts-%.

<u>Beispiel 1:</u> Herstellung von 2,2-Bis-(4-glycidyloxy-3,5-dibromphenyl)-propan. 544 g (2 Aequivalente) 2,2-Bis-(4-hydroxy-3,5-dibromphenyl)-propan (Tetrabrombisphenol A) werden in einem Reaktionsgefäss mit 92,5 g (1 Aequivalent) im Handel erhältlichen Epichlorhydrin, 44 g Wasser und 3,7 g einer 50 %igen, wässrigen Lösung von Benzyltrimethylammoniumchlorid gerührt. Das Reaktionsgemisch wird bis zum Rückfluss auf 90 - 95°C erhitzt, und dann werden innerhalb von 30 Minuten 185 g (2 Aequivalente) Epichlorhydrin zugegeben. Das Reaktionsgemisch wird während 60 Minuten unter Rückfluss gehalten. Durch Erhöhung der äusseren Temperatur und Anlegen eines ansteigenden Vakuums werden 75 g Epichlorhydrin und 44 g Wasser aus dem Reaktionsgemisch entfernt. Der erhaltene Tetrabrombisphenol A-di-(chlorhydrinäther) wird durch Abstreifen (sogenanntes "Strippen") mit 70 ml Wasser gereinigt. Es wird ein festes, schwach gelbes Produkt mit einem Schmelzpunkt von 44,8 - 46,2°C erhalten.

| | |
|---|---|
| Gehalt an hydrolisierbarem Chlor: | 9,6 - 9,8 % |
| Gesamtchlorgehalt: | 9,7 - 9,8 % |
| Epoxidgehalt: | 0,1 - 0,2 Aequivalente/kg |
| Zersetzungstemperatur: | 190°C |

Der oben erhaltene Tetrabrombisphenol A-di-(chlorhydrinäther) wird dann in 537 g Methylisobutylketon gelöst. Unter Rühren gibt man innerhalb von 120 Minuten 179 g einer 50 %igen wässrigen Natronlauge zu und rührt das Reaktionsgemisch 30 bis 60 Minuten bei 85°C. Nach dem Erkalten wird die wässrige Phase von der organischen Phase abgetrennt und die organische Phase wird mittels azeotroper Destillation von den restlichen Spuren Wasser befreit. Nach Zugabe von 1,3 g Filtrierhilfsmittel und Rühren während 5 Minuten wird die Harzlösung durch den "Seitz Supra 80"-Filter bei einem Druck von 0,98 - 1,96 bar filtriert. Das Methylisobutylketon wird unter Vakuum ausgekreist und das zurückbleibende Harz zweimal mit 30 ml Wasser abgestreift (stripped) und getrocknet.
Ausbeute: 636 g (97 % der Theorie) mit einem Epoxidgehalt von 2,98 Aequivalenten/kg.
Viskosität bei 120°C: 263 mPa•s
Gehalt an hydrolisierbarem Chlor: 0,015 %
Gesamtchlorgehalt: 0,15 %.

Beispiel 2: Herstellung von Hexahydrophthalsäurediglycidylester.

300 g (3,49 Aequivalente) Hexahydrophthalsäure und 339 g (3,67 Aequivalente) im Handel erhältliches Epichlorhydrin werden in einem Reaktionsgefäss unter Rühren auf 95°C erhitzt. Dann werden 11,5 g einer 50 %igen wässrigen Tetramethylammoniumchloridlösung zugegeben, und die Reaktionsmischung wird während 210 Minuten auf 95°C erhitzt. Nach Entfernen von 6,9 g Epichlorhydrin im Vakuum wird der zurückbleibende Hexahydrophthalsäuredi-(chlorhydrinester) mit 100 ml Wasser durch sogenanntes Strippen gereinigt. Es wird eine gelbliche viskose Flüssigkeit mit einem Gehalt an hydrolysierbarem Chlor von 18,4 - 18,9 % und einem Gesamtchlorgehalt von 19,4 - 19,9 %
Der Epoxidgehalt beträgt 0,03 - 0,3 Aequivalente/kg und die Zersetzungstemperatur liegt bei 240°C.

Der oben erhaltene Hexahydrophthalsäuredi-(chlorhydrinester) wird in 600 g Methylisobutylketon gelöst und die Lösung auf 42 - 44°C erhitzt. Unter einem Vakuum von 52 mbar werden 305 g einer 50 %igen wässrigen Natronlauge während 360 Minuten zugegeben, wobei Wasser

azeotrop aus der Reaktionslösung entfernt wird. Nach Beendigung der Zugabe von Natronlauge lässt man die Reaktionslösung unter den gleichen Reaktionsbedingungen noch während 90 Minuten nachreagieren. Zwecks Lösen des entstandenen Natriumchlorids werden unter starkem Rühren 600 ml Wasser zugegeben. Nach 15 Minuten wird die wässrige Phase von der organischen Phase abgetrennt. Die organische Phase wird mit 8 ml 10 %iger wässriger Salzsäure neutralisiert und dann mit 200 ml Wasser gewaschen. Mittels Vakuumdestillation wird das Methylisobutylketon entfernt und das erhaltene Harz dreimal mit 25 ml Wasser abgestreift und dann durch einen "Seitz AS"-Filter filtriert.

Es werden 387 g (78 % der Theorie) einer gelblichen niederviskosen Flüssigkeit mit einem Epoxidgehalt von 5,82 Aequivalenten/kg erhalten.
Viskosität bei 25°C: 951 mPa•s
Gehalt an hydrolysierbarem Chlor: 0,046 %
Gesamtchlorgehalt: 0,29 %.

Beispiel 3: Herstellung von N,N,N',N'-Tetraglycidyl-4,4'-diaminodiphenylmethan

198 g (4,0 Aequivalente) 4,4'-Diaminodiphenylmethan und 180 g Wasser werden in einem Reaktionsgefäss unter Rühren auf 70°C erhitzt. Dann werden bei dieser Temperatur 444 g (4,8 Aequivalente) im Handel erhältliches Epichlorhydrin innerhalb von 90 Minuten zugegeben, wobei die Zugabe in den ersten 30 Minuten mit 4 g pro Minute, in den nächsten 30 Minuten mit 5 g pro Minute und in den letzten 30 Minuten mit 5,67 g pro Minute erfolgt. Anschliessend wird das Reaktionsgemisch noch 70 Minuten bei 70°C stark gerührt. Anschliessend wird das überschüssige Epichlorhydrin mittels Vakuumdestillation entfernt, wobei 45 g Epichlorhydrin zurückgewonnen werden, und das zurückbleibende N,N,N',N'-Tetrachlorhydrin-4,4'-diaminodiphenylmethan wird mit 180 g Wasser durch sogenanntes Strippen gereinigt. Es wird ein festes weisses Produkt mit einem Schmelzpunkt von 105 - 110°C (Zersetzung) erhalten.

Gehalt an hydrolysierbarem Chlor: 20,4 - 20,9 %
Gesamtchlorgehalt: 21,7 - 22,1 %
Epoxidgehalt: 0,8 - 1,2 Aequivalente/kg.

Das oben erhaltene N,N,N',N'-Tetrachlorhydrin-4,4'-diaminodiphenylmethan wird in 500 g Methylisobutylketon gelöst, und nach Erhitzen der Lösung auf 65°C werden 196 g einer 50 %igen wässrigen Natronlauge unter Rühren während 45 Minuten zugegeben. Nach Beendigung der Laugezugabe wird die Lösung noch 10 Minuten weitergerührt. Dann wird die wässrige Phase von der organischen Phase abgetrennt. Bei 38 - 40°C und einem Vakuum von 91 mbar werden zur organischen Phase erneut 196 g einer 50 %igen wässrigen Natronlauge zugegeben. Dabei wird das Wasser azeotrop aus dem Reaktionsgemisch entfernt, während das Methylisobutylketon in das Reaktionsgefäss zurückgeführt wird. Nach Beendigung der Laugezugabe wird das Reaktionsgemisch unter den gleichen Reaktionsbedingungen noch 90 Minuten nachreagieren gelassen. Zur Entfernung des entstandenen NaCl werden zum Reaktionsgemisch 380 g Wasser zugegeben. Nach Abtrennung der wässrigen Phase wird die organische Phase mittels azeotroper Destillation getrocknet und danach durch einen "Seitz Supra 80"-Filter filtriert. Das Methylisobutylketon wird im Vakuum in einem Rotationsverdampfer entfernt und das erhaltene Harz zweimal mit 30 ml Wasser behandelt und getrocknet.
Ausbeute: 409 g (97 % der Theorie) gelb-bräunliches Harz
Epoxidgehalt: 8,72 Aequivalente/kg
Viskosität bei 50°C: 8190 mPa•s
Gehalt an hydrolysierbarem Chlor: 0,0066 %
Gesamtchlorgehalt: 0,52 %.

Beispiel 4: Herstellung von 2,2-Bis-(4-glycidyloxy-3,5-dibromphenyl)-propan.

Beispiel 1 wird unter Verwendung von Tetramethylammoniumchlorid anstelle von Benzyltrimethylammoniumchlorid wiederholt. Der in Zwischenstufe erhaltene Tetrabrombisphenyl A-di-(chlorhydrinäther) weist folgende Kenndaten auf:

| | |
|---|---|
| Gehalt an hydrolisierbarem Chlor: | 9,6 - 9,8 % |
| Gesamtchlorgehalt: | 9,7 - 9,9 % |
| Epoxidgehalt: | 0,1 - 0,2 Aequivalente/kg |
| Zersetzungstemperatur: | 190°C. |

Patentansprüche

1. Verfahren zur Herstellung von Glycidyläthern der Formel I

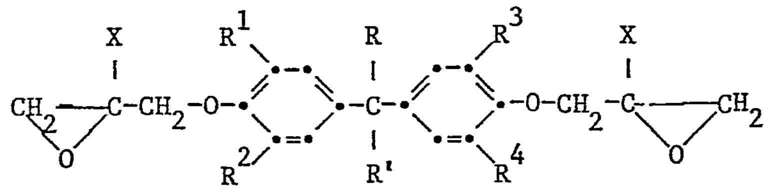

(I) ,

worin R und R' unabhängig voneinander je ein Wasserstoffatom, Alkyl mit 1 bis 15 C-Atomen, Phenyl, Cyclohexyl oder Cyclopentyl, $R^1$, $R^2$, $R^3$ und $R^4$ unabhängig voneinander je ein Chlor- oder Bromatom und beide X je ein H-Atom oder Methyl bedeuten, von Glycidylestern der Formel II

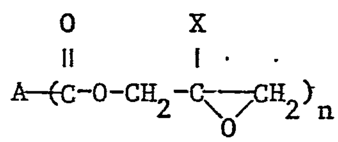

(II) ,

worin n 1 oder 2 bedeutet, A, wenn n 1 ist, für einen einwertigen aliphatischen Rest oder, wenn n 2 ist, für eine direkte Bindung oder einen zweiwertigen aliphatischen, aromatischen oder cycloaliphatischen Rest und X für ein H-Atom oder Methyl stehen, von N-Glycidylverbindungen der Formel III

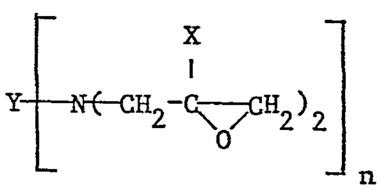

(III) ,

worin n 1 oder 2 bedeutet, Y für einen n-wertigen aliphatischen oder aromatischen Rest und X für ein H-Atom oder Methyl stehen, von Triglycidylisocyanurat oder Tri-(β-methylglycidyl)-isocyanurat durch Umsetzung eines Epihalogenhydrins mit einer Verbindung der Formeln Ia, IIa oder IIIa

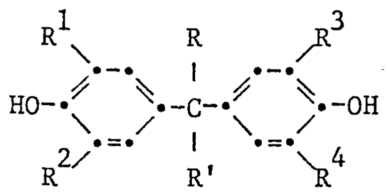 (Ia) ,

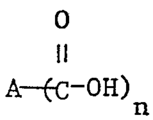 (IIa) , $Y-(NH_2)_n$ (IIIa) ,

oder mit Cyanursäure, gegebenenfalls im wässrigen Medium und/oder in Gegenwart eines in Wasser beständigen Katalysators, zu den entsprechenden Halogenhydrinverbindungen und anschliessende Dehydrohalogenierung mit wässrigem Alkali zu den Glycidylverbindungen der Formeln I, II bzw. III oder zu Triglycidyl- bzw. Tri(β-methylglycidyl)-isocyanurat, dadurch gekennzeichnet, dass man auf 1 Aequivalent reaktives H-Atom in den Verbindungen der Formeln Ia bis IIIa oder in der Cyanursäure mehr als 1 und höchstens 1,8 Aequivalente Epihalogenhydrin einsetzt, die in erster Stufe erhaltenen Halogenhydrinverbindungen mittels azeotroper Destillation vom überschüssigen Epihalogenhydrin befreit und dann durch sogenanntes Strippen mit Wasser wäscht und in zweiter Stufe die Dehydrohalogenierung der Halogenhydrinverbindungen in einem inerten, im wesentlichen mit Wasser nicht mischbaren organischen Lösungsmittel vornimmt.

2. Verfahren gemäss Anspruch 1, dadurch gekennzeichnet, dass man auf 1 Aequivalent reaktives H-Atom 1,05 bis 1,5 Aequivalente Epihalogenhydrin einsetzt.

3. Verfahren gemäss Anspruch 1, dadurch gekennzeichnet, dass man die Umsetzung des Epihalogenhydrins mit einer Verbindung der Formel Ia - IIIa oder mit Cyanursäure in wässrigem Medium durchführt.

4. Verfahren gemäss Anspruch 1, dadurch gekennzeichnet, dass man

die Umsetzung des Epihalogenhydrins mit einer Verbindung der Formel Ia- IIIa oder mit Cyanursäure in Gegenwart eines in Wasser beständigen Katalysators vornimmt.

5. Di-(halogenhydrinäther) der Formel V

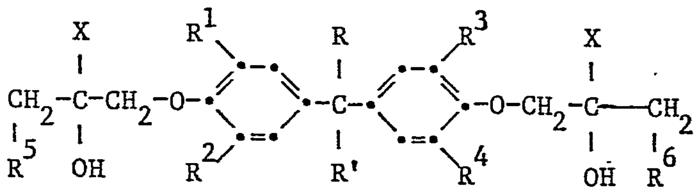

(V) ,

worin R und R' unabhängig voneinander je ein Wasserstoffatom, Alkyl mit 1 bis 15 C-Atomen, Phenyl, Cyclohexyl oder Cyclopentyl, beide X je ein Wasserstoffatom oder Methyl, $R^1$, $R^2$, $R^3$, $R^4$, $R^5$ und $R^6$ je ein Chlor- oder Bromatom bedeuten.

6. Di-(halogenhydrinäther) der Formel V gemäss Anspruch 5, worin R und R' je für Methyl, beide X je für ein Wasserstoffatom, $R^1$, $R^2$, $R^3$ und $R^4$ je für ein Bromatom und $R^5$ und $R^6$ je für ein Chloratom stehen.